# EUROPEAN PATENT SPECIFICATION

(11) **EP 3 851 106 B1**
(45) Date of publication and mention of the grant of the patent: **11.01.2023**
(21) Application number: 19867766.8
(22) Date of filing: 05.06.2019
(51) Int. Cl.: A61K 31/497, C07D 401/14, A61P 7/00, A61P 7/10, A61P 9/00, A61P 9/02, A61P 9/12, A61P 39/00, A61P 43/00

(54) **PROSTACYCLIN RECEPTOR AGONIST FOR USE IN TREATING OR PREVENTING ALTITUDE SICKNESS**
PROSTACYCLINREZEPTORAGONIST ZUR BEHANDLUNG ODER VORBEUGUNG DER HÖHENKRANKHEIT
AGONISTE DU RÉCEPTEUR DE LA PROSTACYCLINE DESTINÉE AU TRAITEMENT OU À LA PREVENTION DU MAL DES HAUTEURS

(30) Priority: 25.09.2018 CN 201811113332
(43) Date of publication of application: 21.07.2021
(73) Proprietor: Chinese PLA General Hospital, Beijing 100039 (CN); Shijiazhuang Sagacity New Drug Development Company, Ltd., Hi-Tech District Shijiazhuang Hebei 050035 (CN)
(72) Inventor: HE, Kunlun, Beijing 100039 (CN); ZHANG, Zeyu, Beijing 100039 (CN); GAO, Xiaojian, Beijing 100039 (CN); LIU, Chunlei, Beijing 100039 (CN); LI, Xin, Beijing 100039 (CN); LI, Chen, Beijing 100039 (CN); QIAN, Wenyuan, Shanghai 200131 (CN); YANG, Chundao, Shanghai 200131 (CN); XU, Guanghai, Shanghai 200131 (CN); WANG, Yiwei, Hebei 100039 (CN)
(74) Representative: Murgitroyd & Company
(86) International application number: PCT/CN2019/090110
(87) International publication number: WO 2020/062913

(56) References cited:
- EP-A1- 3 750 879

## Description

The present disclosure relates to the technical field of biological medicine, and particularly relates to a prostacyclin receptor agonist sc0253 for use in treating or preventing a high altitude sickness.

### BACKGROUND

High altitude sickness, i.e., a high altitude disease, is a natural physiological reaction generated by the body for adapting to changes of atmospheric pressure difference, low oxygen content, dry air and the like caused by the altitude after a person reaches a certain altitude. Symptoms of the high altitude sickness generally include headache, palpitation, fatigue, chest stuffiness, shortness of breath, emesis, appetite decrease, twitch, confusion, cognitive ability plummet and the like. Physical signs include cardiac acceleration, deepening breath, mild abnormal blood pressure, face or limb edema, paro xymally cyanosis and the like. Currently, drugs such as root of kirilow rhodiola, GaoYuanning, American ginseng, radix salviae miltiorrhizae pills, Bufferin and the like or related health care products are mostly adopted to prevent and condition the high altitude sickness. For example, there are patents CN103829245A, CN103948896A, CN104274808A, CN104288262A, CN104288735A, CN104288476A, CN104721202A, CN104706771A, CN105168308A, CN105193839A and the like, but these drugs or foods have defects of slow response, many side effects and the like.

The inventor is amazed to find a type of prostacyclin (PGI₂) receptor agonist compounds which have remarkable curative effects in the aspect of treating or preventing the high altitude disease.

The patent WO2002/88084 discloses a medicinal composition using a compound serving as a PGI₂ receptor agonist as an active ingredient and medical application in the aspects of inhibiting platelet aggregation, inhibiting the binding of ³H-Iloprost to a platelet membrane and improving the effect of cAMP in platelets.

The patent WO2010/150865 discloses a crystal. The crystal is the PGI₂ receptor agonist. A medicinal composition using the crystal as an active ingredient can treat or prevent the transient ischemic attack, diabetic neuropathy, diabetic gangrene, peripheral circulatory disorder, the collagen disease, reocclusion or restenosis after percutaneous transluminal coronary angioplasty, i.e., PTCA, arteriosclerosis, thrombosis, hypertension, pulmonary arterial hypertension, the ischemic disease, angor pectoris, glomerulonephritis, diabetic nephritis, the chronic renal failure, allergy, bronchial asthma, ulcers, decubitus, restenosis after percutaneous coronary intervention such as atherectomy, stent implantation and the like, thrombocytopenia caused by dialysis, diseases related to fibrosis of organs or tissues, erectile dysfunction, the inflammatory bowel disease, gastritis, the gastric ulcer, ischemic ophthalmopathy, sudden deafness, avascular bone necrosis, the intestinal injury generated along with drug administration of non-steroid anti-inflammatory drugs, i.e., NSAIDs, symptoms generated along with spinal stenosis and the like. But application of the compound disclosed by the present disclosure to preparation of a medicinal composition for treating or preventing the high altitude disease is not disclosed.

### SUMMARY

The present disclosure provides a compound, or a pharmaceutically acceptable salt thereof, for use in treating or preventing high altitude sickness, wherein the compound is sc0253 having the following structure:

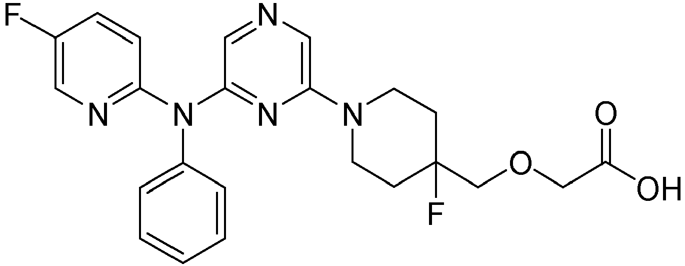

Preferably, the clinical manifestation of the high altitude sickness is selected from one or a combination of two or more of headache, vertigo, palpitation, cardiac acceleration, fatigue, chest stuffiness, shortness of breath, deepening breath, nausea, emesis, insomnia, weakness, giddiness, somnolence, appetite decrease, twitch, confusion, numbness of hands and feet, paroxymally cyanosis, face edema, limb edema or cognitive ability plummet.

According to the present disclosure, a medicinal composition includes the compound which is used as an active ingredient, or the pharmaceutically acceptable salt of the compound, and a medicinal auxiliary material.

Preferably, the medicinal auxiliary material is selected from one or a combination of two or more of a solvent, an emulsifier, a plasticizer, a disintegrant, a filling agent, an adhesive, a sweetening agent or a lubricant.

According to the present disclosure, the solvent is selected from one or a combination of two or more of water, alcohols, phenols, ethers, halogenated hydrocarbon, ketones, aldehydes, nitriles, amide, sulfone, hydroxypropyl-beta-cyclodextrin (HP-β-CD), sulfoxide or carboxylic acid.

Preferably, the solvent is selected from one or a combination of two or more of methanol, ethanol, isopropanol, hydroxypropyl-beta-cyclodextrin, polyoxyl-15-hydroxystearate, dichloromethane acetone or ethyl acetate.

According to the present disclosure, the emulsifier is selected from one or a combination of two or more of polyethylene glycol oleate, polyvinyl alcohol, glyceryl stearate or tween-80. Preferably, the emulsifier is selected from one or a combination of the polyethylene glycol oleate or the glyceryl stearate.

According to the present disclosure, the plasticizer is selected from one or a combination of two or more of polyethylene glycol, castor oil, glycerin or sorbitol. Preferably, the plasticizer is selected from one or a combination of the polyethylene glycol or the castor oil.

According to the present disclosure, the disintegrant is selected from one or a combination of two or more of crosslinked povidone, sodium hydroxymethyl cellulose, sodium methyl cellulose starch or low-substituted hydroxypropyl cellulose. Preferably, the disintegrant is selected from one or a combination of the sodium hydroxymethyl cellulose or the sodium methyl cellulose starch.

According to the present disclosure, the filling agent is selected from one or a combination of two or more of microcrystalline cellulose, erythritol, sorbitol, mannitol, pregelatinized starch, calcium carbonate, sucrose or lactose. Preferably, the filling agent is selected from one or a combination of two or more of the sorbitol, the mannitol, the pregelatinized starch or the lactose.

According to the present disclosure, the adhesive is selected from one or a combination of two or more of polyvinylpyrrolidone, carbomer, hydroxypropyl cellulose, gelatin, guar gum, sodium hydroxymethyl cellulose, hydroxypropyl methylcellulose, magnesium aluminosilicate, ethyl cellulose, hydroxyethyl cellulose, pregelatinized starch, Arabic gum, polyvinyl alcohol, povidone, maltodextrin or sodium alginate. Preferably, the adhesive is selected from one or a combination of two or more of the magnesium aluminosilicate, the sodium hydroxymethyl cellulose or the polyvinylpyrrolidone.

According to the present disclosure, the sweetening agent is selected from one or a combination of two or more of aspartame, xylitol, menthol, peppermint essence, acesulfame potassium, steviol glycosides or sucralose. Preferably, the sweetening agent is selected from one or a combination of two or more of the peppermint essence, the sucralose or the acesulfame potassium.

According to the present disclosure, the lubricant is selected from one or a combination of two or more of talcum powder, hydrogenated calcium stearate, magnesium dodecyl sulfate, sodium stearyl fumarate, hydrated sodium silica gel, hydrogenated castor oil, zinc stearate or magnesium stearate. Preferably, the lubricant is selected from one or a combination of two or more of the hydrogenated castor oil, the zinc stearate or the hydrogenated calcium stearate.

Preferably, the medicinal composition is applied into a body in oral administration, intravenous or intraperitoneal ways.

In a specific embodiment of the present disclosure, the medicinal composition is applied into the body in the oral administration way.

Preferably, a dosage form of the medicinal composition is one or a combination of two or more of oral liquid, pills, granules, tablets or capsules.

According to the present disclosure, the high altitude environment has an altitude of 2,000m or above, and has conditions of low pressure and shortage of oxygen. Preferably, the high altitude environment has an altitude of 2,700m or above, and has conditions of low pressure and shortage of oxygen.

In a specific embodiment of the present disclosure, the high altitude environment has an altitude of 5,500m or above, and has conditions of low pressure and shortage of oxygen.

According to the present disclosure, the "active ingredient" refers to a chemical entity, and can treat target disorder, diseases or conditions.

According to the present disclosure, the "and/or" includes one listed item and a combination of any number of items.

According to the present disclosure, the "treatment" denotes that after a disease has begun to develop, the progress or severity of a type of physical sign, symptom, disorder, condition or disease is retarded, interrupted, prevented, controlled, stopped, relieved or reversed, but it does not necessarily involve complete elimination of related physical signs, symptoms, conditions or disorder of all diseases.

According to the present disclosure, the "or pharmaceutically acceptable salt thereof" refers to a salt prepared from pharmaceutically acceptable non-toxic acid or alkali, wherein the acid or the alkali includes inorganic acid or alkali or organic acid or alkali. The inorganic acid is selected from hydrochloric acid, hydrobromic acid, phosphoric acid, hydroiodic acid or sulfuric acid. The inorganic alkali is selected from calcium, magnesium, lithium, sodium, zinc, aluminium or potassium. The organic acid is selected from formic acid, glycolic acid, propionic acid, acetic acid, succinic acid, methane sulfonic acid, ethanesulfonic acid, maleic acid, glutamic acid, benzoic acid, stearic acid, alginic acid, benzene sulfonic acid, glucuronic acid, pamoic acid or galacturonic acid. The organic alkali is selected from diethanolamine, choline, procaine, lysine or 1,2-ethylenediamine.

### BRIEF DESCRIPTION OF THE DRAWINGS

The embodiments of the present disclosure will be illustrated in detail below in connection with the drawings, wherein:

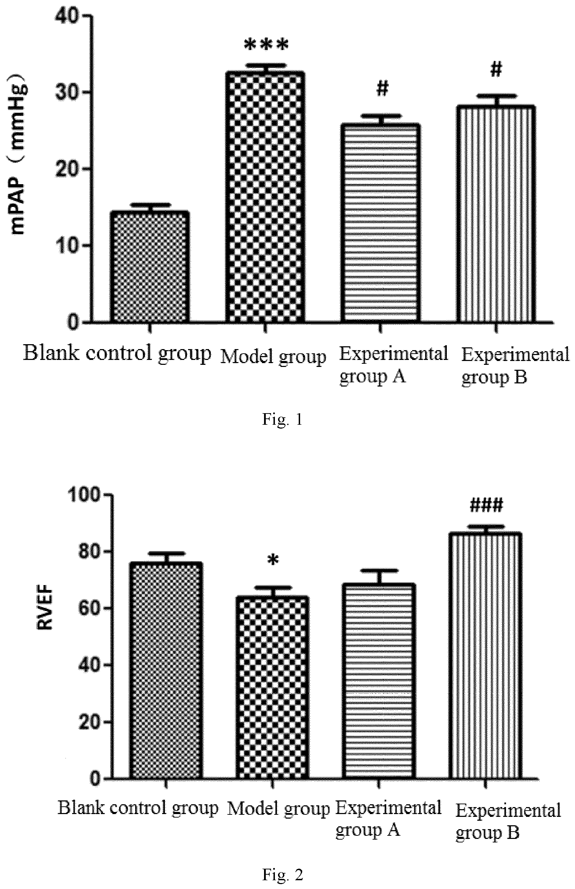

Fig. 1 shows a change of a mean pulmonary arterial pressure (mPAP) of rats in each group (a blank control group, a model group, an experimental group A and an experimental group B);
Fig. 2 shows a change of a right ventricular ejection fraction (RVEF) of rats in each group (the blank control group, the model group, the experimental group A and the experimental group B);

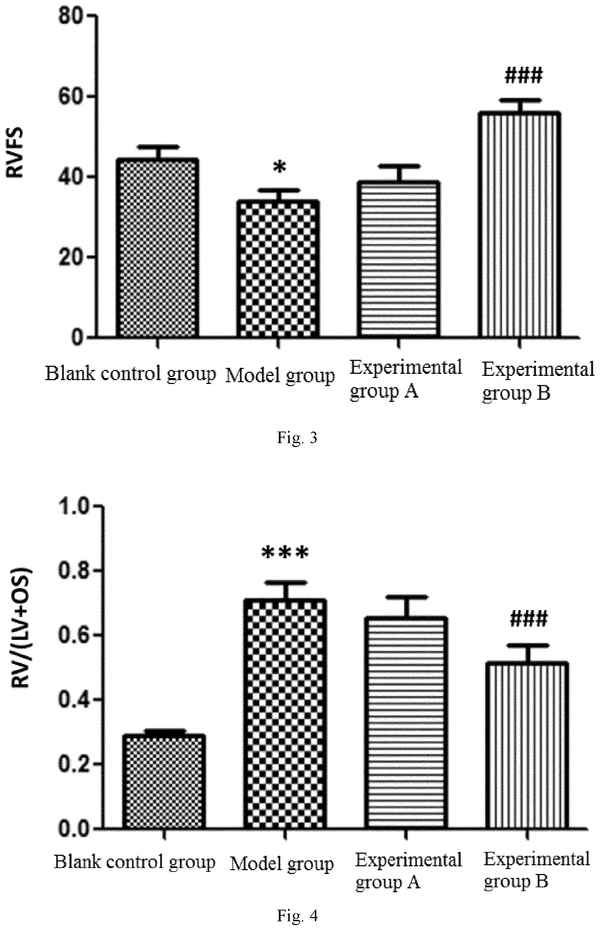

Fig. 3 shows a change of right ventricular fractional shortening (RVFS) of rats in each group (the blank control group, the model group, the experimental group A and the experimental group B); and
Fig. 4 shows a change of a right ventricular hypertrophy index (RV/(LV+OS)) of rats in each group (the blank control group, the model group, the experimental group A and the experimental group B).

### DETAILED DESCRIPTION

The technical solutions in the embodiments of the present disclosure will be described in a clearly and fully understandable way in connection with the drawings in the embodiments of the present disclosure.

### Embodiment 1

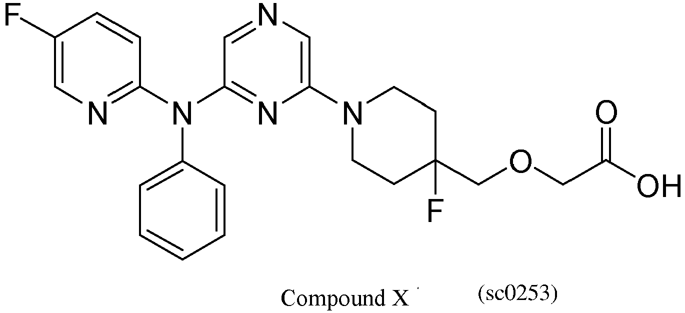

### S1: Synthesis of compound A

A synthesis route of a compound A is as follows: 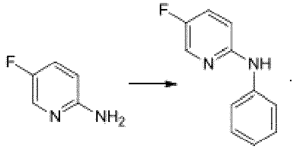 2-amino-5-fluoropyridine (2g, 17.84mmol), phenylboronic acid (4.35g, 35.68mmol), copper acetate (3.24g, 17.84mmol) and pyridine (2.82g, 35.68mmol) are added into dichloromethane (20mL), and after reaction liquid is stirred for 14 hours at the temperature of 25 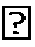 in an oxygen atmosphere (15psi), concentration is carried out to obtain a crude product. The crude product is subjected to column separation (an eluent: petroleum ether/ethyl acetate=25:1) to obtain the compound A. MS *m*/*z*: 189.0[M+H]+.

### S2: Synthesis of compound B

A synthesis route is as follows:

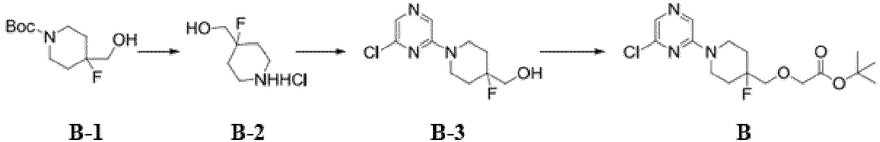

A compound B-1 (2g, 8.57mmol) is uniformly mixed with ethyl acetate hydrochloride (10.00mL), a mixed solution is stirred for 0.5 hour at the temperature of 20°C, and after the reaction is finished, reaction liquid is filtered to obtain a compound B-2.

The compound B-2 (3g) and piperidyl methanol (2.44g, 21.14mmol) as well as triethylamine (2.24g, 22.15mmol, 3.08mL) are added into dioxane (60.00mL), uniform mixing is carried out, and reaction liquid is stirred for 2 hours at the temperature of 105°C. After the reaction is finished, a solvent is removed under reduced pressure, and the obtained residue is subjected to column chromatography separation (a developing agent: petroleum ether/ethyl acetate = 10:1 to 3:1) to obtain a compound B-3.

The compound B-3 (3g) and tetrabutylammonium hydrogen sulfate (4.47g, 13.18mmol) are dissolved into methylbenzene (60.00mL), the temperature is reduced to 0°C, stirring is carried out for 10 minutes, after a 40% potassium hydroxide solution (60mL) is added into reaction liquid and stirring is carried out for 20 minutes, tert-butyl bromoacetate (7.71g, 39.53mmol, 5.84mL) is added into the reaction liquid, and the reaction liquid is stirred for 12 hours at the temperature of 30°C. The obtained product is poured into water (20mL), extraction is carried out by ethyl acetate (30mL), and a separated organic phase is washed with a saturated salt solution (20mL) and dried by anhydrous sodium sulfate. After filtering is carried out to remove a desiccant, a solvent is removed under reduced pressure, and the obtained residue is subjected to column chromatography separation (a developing agent: ethyl acetate/petroleum ether = 1:4) to obtain the target compound B. MS *m*/*z*: 360.0 [M+H]+. 1H NMR (400MHz, METHANOL-d4) δ ppm 8.16 (s, 1H), 7.74 (s, 1H), 4.24 (br d, *J*=13.6 Hz, 2H), 4.07 (s, 2H), 3.63 (d, *J*=19.2 Hz, 2H), 3.37 (d, *J*=3.0 Hz, 1H), 3.30 (d, *J*=3.0 Hz, 1H), 2.04-1.73 (m, 4H), 1.50 (s, 9H).

### S3: Synthesis of compound C

The compound B (0.1g), cesium carbonate (285.95mg, 877.62µmol), xantphos (33.85mg, 58.51µmol) and Pd(dba)2 (16.82mg, 29.25µmol) are added into a dioxane solution (10mL) of the compound A (54.77mg), and a reaction system is stirred for 12 hours at the temperature of 100°C under the protection of nitrogen gas. Water (20mL) is added into the reaction system for dilution, and extraction is carried out by ethyl acetate (10mL^{∗}3). A mixed organic phase is washed with a saturated salt solution (20mL) and dried by anhydrous sodium sulfate. Filtering is carried out, and the obtained filtrate is concentrated to obtain a crude product. The crude product is subjected to preparative thin layer chromatography (a ratio of petroleum ether to tetrahydrofuran is 2:1) separation to obtain a compound C. MS *m*/*z*: 512.2 [M+H]+.

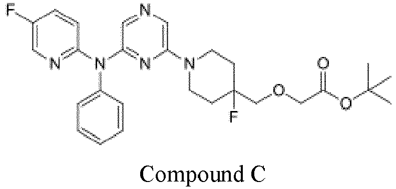

### S4: Synthesis of compound X (sc0253)

10% sodium hydroxide (8mL) is added into a methanol (10mL) solution of the compound C, and a reaction system is stirred for 0.5 hour at the temperature of 45°C. Reaction liquid is concentrated, diluted by water (20mL) and stirred for 2 minutes, pH of the reaction system is regulated into 5 by diluted hydrochloric acid (2N), and extraction is carried out by ethyl acetate (30mL^{∗}2). A mixed organic phase is washed with a saturated salt solution (20mL), dried by anhydrous sodium sulfate and filtered, and the obtained product is concentrated under the vacuum condition to obtain a crude product. The crude product is subjected to high performance liquid chromatography (HPLC) (neutral) separation to obtain the compound X. MS *m*/*z:* 456.0 [M+H]+. 1H NMR (CHLOROFORM-d, 400MHz) δ ppm 8.21 (d, *J*=2.4 Hz, 1H), 7.70 (s, 1H), 7.43-7.35 (m, 4H), 7.28-7.24 (m, 1H), 7.19 (d, *J*=7.2 Hz, 2H), 7.10 (dd, *J*=3.6, 8.8 Hz, 1H), 4.14 (s, 2H), 3.90 (d, *J*=13.6 Hz, 2H), 3.58 (br d, *J*=19.2 Hz, 2H), 3.19 (t, *J*=12.0 Hz, 2H), 1.92-1.86 (m, 2H), 1.76-1.59 (m, 2H).

### Embodiment 2

### I Material and method

1. Experimental animals and feeding
   40 SD rats (about 200g, male, clean) are purchased from Beijing Vital River Laboratory Animal Technology Co. Ltd and have the license number of SCXK(Jing)2016-0006. The SD rats are fed in a low-pressure oxygen cabin, and regularly fed with a complete nutritional feed under the conditions of the room temperature of 22 to 25°C and the humidity of 30% to 50%.
2. Reagents and sample groups
   A compound sc0253 is the compound X prepared in Embodiment 1 of the present disclosure;
   HP-β-CD is purchased from solaxbio;
   Solvent configuration: 20% of HP-β-CD and 80% of double distilled water, and pH = 8.
   Groups:
      A blank control group: normal-pressure normal-oxygen feeding
      A model group: a low-pressure low-oxygen cabin, and intragastric administration of a solvent
      An experimental group A: a low-pressure low-oxygen cabin, and intragastric administration of sc0253 (5mg/kg) as well as a solvent
      An experimental group B: a low-pressure low-oxygen cabin, and intragastric administration of sc0253 (10mg/kg) as well as a solvent
3. Instruments
   A multi-factor composite environment simulated medical science experiment module (the type of DYC-3285, the Instrument Center of the Beijing Military Medical Science Academy);
   A small animal breathing machine (Kent scientific, the United States);
   A multifunctional physiograph (Millar, the United States);
   A small animal ultrasonic instrument (Visual Sonics Inc, Canada).
4. Experiment Design and Process
   40 rats are randomly divided into four groups, and each group includes 10 rats. 3 groups are placed into an experiment module, the pressure inside the module is regulated to 380mmHg, a high altitude environment with an altitude of 5,500 meters is simulated, the experiment module is opened for 1 hour every day so as to add feed and water for animals and carry out corresponding medicine treatment, meanwhile, the environment where the rats are positioned are kept alternate day and night according to a ratio of 12h:12h, and after 14 days, intragastric administration is respectively carried out in which the solvent (the model group), the sc0253 (5mg/kg) and the solvent (the experimental group A) or the sc0253 (10mg/kg) and the solvent (the experimental group B) are respectively applied, and the operation is carried out twice every day and continued for 14 days. Rats in the fourth group are placed in the same room to be fed in the normal-pressure normal-oxygen environment (the blank control group).
5. Index detection method
   3% pentobarbital sodium (0.2mL/100g) is intraperitoneally injected to anesthetize the rats, and ultrasonic detection is carried out. The following ultrasonic data is recorded: right ventricular ejection fraction (RVEF); and right ventricular fractional shortening (RVFS). Then the anesthetized rats are fixed on an operating table in a supine position mode, tracheotomy is carried out, a breathing machine is connected, thoracotomy is carried out to expose the hearts, a catheter is inserted into each right ventricle, right heart catheterization is carried out, and the right ventricular systolic pressure is recorded. Then each catheter is slowly pushed forwards, and can reach a corresponding pulmonary artery through a corresponding right ventricular outflow tract, the pressure waveform of a monitor is observed, and the mean pulmonary arterial pressure mPAP is recorded. The rats are executed, the hearts are taken out, the atrial tissue and attached fat are removed, left and right ventricles are separated, moisture is sucked up by filter paper, the weights of the right ventricles and the left ventricles are respectively weighed, and (RV/(LV+IS)) is calculated.
6. Statistical method
   All the data is represented by x±s, comparison among the groups is single factor analysis of variance, when P<0.05, it represents that the difference has a statistical significance, and statistical treatment is carried out by adopting an SPSS19.0 software package.

### II Experimental result

1. Influence on rat mPAP
   After the rats are fed for 14 days in a low-pressure low-oxygen environment, compared with the blank control group, the mPAP of the rats in the model group is obviously increased and the difference has significance (P<0.001). In the low-pressure low-oxygen environment, compared with the model group, the mPAP of the rats in the experimental group A (the compound sc0253-5mg/kg) and the experimental group B (sc0253-10mg/kg) is obviously reduced and the difference has statistical significance (P<0.05).
2. Influence on rat RVEF and RVFS
   After the rats are fed for 14 days in the low-pressure low-oxygen environment, compared with the blank control group, the RVEF and the RVFS of the rats in the model group are obvious reduced and the differences have significance (P<0.05). In the low-pressure low-oxygen environment, compared with the model group, the RVEF and the RVFS of the rats in the experimental group B (the compound sc0253-10mg/kg) are obviously increased and the differences have statistical significance (P<0.01).
3. Influence on rat RV/(LV+OS)
   After the rats are fed for 14 days in the low-pressure low-oxygen environment, compared with the blank control group, the RV/(LV+OS) of the rats in the model group is obvious increased and the difference has significance (P<0.01). In the low-pressure low-oxygen environment, compared with the model group, the RV/(LV+OS) of the rats in the experimental group B (the compound sc0253-10mg/kg) is obviously reduced and the difference has statistical significance (P<0.01).

## Claims

1. A compound 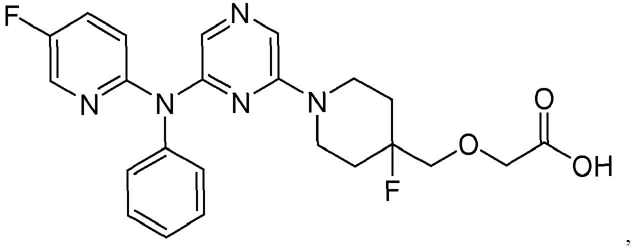 or a pharmaceutically acceptable salt thereof for use in treating or preventing high altitude sickness.

## Patentansprüche

1. Eine Verbindung 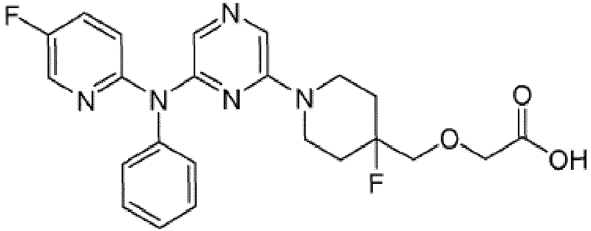 oder ein pharmazeutisch akzeptables Salz davon zur Verwendung bei der Behandlung oder Vorbeugung von Höhenkrankheit.

## Revendications

1. Un composé 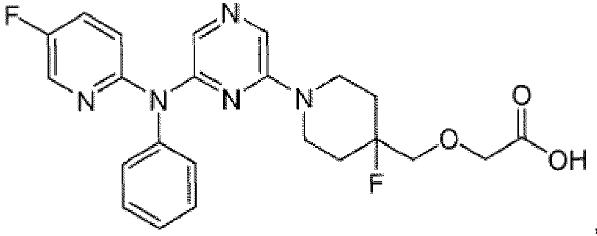 , ou un sel pharmaceutiquement acceptable de celui-ci pour une utilisation dans le traitement ou la prévention du mal de haute altitude.
